# EUROPÄISCHE PATENTSCHRIFT

(11) **EP 1 199 990 B1**
(45) Veröffentlichungstag und Bekanntmachung des Hinweises auf die Patenterteilung: **22.09.2004**
(21) Anmeldenummer: 00947899.1
(22) Anmeldetag: 30.06.2000
(51) Int. Cl.: A61B 17/122, A61B 17/128, A61B 17/068

(54) **APPLIKATORSPITZE EINES CHIRURGISCHEN APPLIKATORS ZUM SETZEN VON CLIPS/KLAMMERN**
SURGICAL APPLICATOR TIP FOR DELIVERING CLIPS/CLAMPS
POINTE D'APPLICATEUR CHIRURGICAL DESTINEE A LA POSE D'AGRAFES ET D'ATTACHES

(30) Priorität: 30.07.1999 DE 19935904
(43) Veröffentlichungstag der Anmeldung: 02.05.2002
(73) Patentinhaber: Forschungszentrum Karlsruhe GmbH, 76133 Karlsruhe (DE)
(72) Erfinder: KNEIFEL, Bernhard, D-76767 Hagenbach (DE); HERRMANN, Günter, D-67269 Grünstadt (DE)
(86) Internationale Anmeldenummer: PCT/EP2000/006110
(87) Internationale Veröffentlichungsnummer: WO 2001/008567

(56) Entgegenhaltungen:
- EP-A- 0 094 752
- EP-A- 0 592 244
- WO-A-87/04063
- US-A- 3 082 426
- US-A- 5 915 615

## Beschreibung

Die Erfindung betrifft eine Applikatorspitze eines chirurgischen Applikators zum Setzen von gewebeverbindenden Clips/Klammern.

Mit Hilfe von minimal invasiven Operationsgeräten lassen sich Eingriffe ohne größere Schnitte und Narben bewerkstelligen. Mit einem Applikator werden Gewebeteile unter Anwendung von Clips/Klammern verbunden. Man verwendet sie beispielsweise beim Operieren von Brüchen, indem Netze zur Stabilisierung mit dem Gewebe verbunden werden. Zur Zeit sind Clips aus Titan im Einsatz, die biologisch nicht abbaubar sind, also im Körper verbleiben oder später u. U. bei Beschwerden operativ entfernt werden müssen: Angestrebt wird für die Verankerung während der Wundheilungszeit, Clips einzusetzen, die während dieser Zeit eine notwendige mechanische Festigkeit durch den biologischen Abbau, Resorbtion durch den Körper, nicht unterschreiten, aber auf längere Sicht völlig abgebaut werden.

Solche Clips haben eine spezielle krallenförmige Gestalt, die sich unter vorübergehender Krafteinwirkung zangenartig schließen und in dieser Lage stabil verbleiben. Hierzu ist ein Applikator notwendig, mit dem solche Clips zuverlässig in einem einfachen Bedienvorgang gesetzt werden können.

Eine Applikatorspitze mit den Merkmalen des ersten Teils des Anspruchs 1 ist aus EP-A-0 592 244 bekannt.

Der Erfindung liegt also die Aufgabe zugrunde, die Spitze eines chirurgischen Applikators zum Setzen von Clips/Klammern derart zu gestalten, daß einerseits ausreichend Clips/Klammern dort gestapelt, magaziniert, werden können, und andrerseits ein zur Entnahme positionierter Clip/positionierte Klammer sicher entnommen und komplikationslos in Position zum Klammern gebracht werden kann.

Der Applikator besteht im Prinzip aus drei Hauptkomponenten: dem Bediengriff, dem Rohr, und der Applikatorspitze. Der Bediengriff dient dem Halten des Applikators und nimmt die Einrichtungen zum Drehen, Schwenken der Spitze, sowie das Auslösen der Clips auf. Am Griff ist ein Rohr befestigt, in dem die Übertragungselemente zur Betätigung der Spitze laufen. Am Ende des Rohrs ist die bewegliche und drehbare Applikatorspitze angebracht. Sie dient der Magazinierung der Clips, sowie deren Fördern, Einstechen und Verschließen. Der Trokar führt den Applikator z.B. durch die Bauchdecke und dichtet zusätzlich noch den unter Druck stehenden Bauchraum gegen die Umgebung ab.

Die Aufgabe wird durch die konstruktive Gestaltung der Spitze eines Applikators gemäß den kennzeichnenden Merkmalen des Anspruchs 1 gelöst. Die Applikatorspitze dient der Aufnahme der Clips, sowie der peripheren Mechanik. Zusätzlich ist die Spitze dreh- und schwenkbar. Die Clips haben eine spezielle Form: Sie sind krallenartig, haben, auf einer Stapelstange aneinander gereiht, eine gerade Schulter, die durch einen Kniehebel überbrückt ist. Der vorderste Clip liegt an einer Klemmkante oder Anschlagkante am freien Ende der Stange an, die nachfolgenden reihen sich über Federkraft an, so daß beim Abheben des vordersten die verbliebene Reihe nachrückt. Die Stapel- oder Magazinstange ist mit ihrer hinteren Stirn an einer Basisplatte verankert, an deren Seite der Magazindeckel drehbar ansetzt. Die Basisplatte wird beim Einbringen in den Magazinschacht in einer Ausnehmung verankert und über Federkraft in Position gehalten.

Unter den magazinierten Clips befindet sich die axial bewegliche Mechanik in Form der beiden Stempel, dem Förder- und Schließstempel. Beide werden durch einen im Applikatorschaft geführten Draht über die Betätigung am Bediengriff axial angetrieben. Bei der Vorwärtsbewegung wird der Schließstempel zunächst funktionslos mitgenommen, der Förderstempel entnimmt aber mit seinen beiden Mitnehmerzähnen schon den Clip an der Front des Magazins und führt ihn unter einer überlagerten 90°-Drehung einsetzgerecht an die Operationsöffnung der Applikatorspitze. Dort wird er in Position verankert, gleichzeitig legt sich der Schließstempel über eine schräge Rampe an das Kniegelenk des Kniehebels an.

Weitere zweckmäßige konstruktive Maßnahmen sind in den Unteransprüchen 2 bis 6 gekennzeichnet. So kann die Applikatorspitze durch den Magazindeckel im Mantelbereich verschlossen werden, der Deckel rastet durch eine kurze axiale Bewegung in ein Schloß in der Wand ein und wird dort über eine Feder in dieser Position gehalten (Anspruch 2).

Der Förderstempel wird gewissermaßen unter den im Magazin hängenden Clips vorgeschoben und hebt mit seinen beiden vorgestreckten Mitnehmerzähnen den vordersten an seinem zylindrischen Schulterfortsatz ab (Anspruch 3).

Die Führungsrinne, in der der zu Beginn des Vorschiebens um 90° gedrehte Clip geführt wird, weist rückwärtssperrende Federeinrichtungen (Anspruch 4) auf.

Ist der Clip am Ausgang der Applikatorspitze angekommen, wird er dort zunächst über eine Rückhalteklammer in Position gehalten (Anspruch 5), bis er über das Vorschieben des Schließstempels in das Gewebe zangenartig eingedrückt und durch das Durchdrücken des Kniehebels in die gestreckte stabile Lage darin geschlossen wird (Anspruch 6).

Die Applikatorspitze hat einen Magazinschacht, in dem eine ausreichend große Anzahl an Clips, weil hängend aneinander gereiht, untergebracht werden können. Die Förder- und Schließmechanik wird über eine Antriebseinrichtung bedient. Sie ist konstruktiv einfach und damit zuverlässig aufgebaut. Die Gestaltung des Innenraums der Applikatorspitze erlaubt eine sichere Führung und abschließende Positionierung des Clips für das zielsichere Setzen im zu verklammernden Gewebe.

Die Applikatorspitze wird im folgenden anhand der Zeichnung näher erläutert und die Bedienung beschrieben. Die Zeichnung besteht aus den Figuren 1 bis 9. Es zeigt:
Figur 1 das Magazin für die Clips,
Figur 2 die leere Applikatorspitze mit Mechanik,
Figur 3 das im Magazinschacht eingerastete und verschlossene Magazin,
Figur 4 das Ansetzen des Förderstempels am Clip,
Figur 5 das Vorschieben des Clip,
Figur 6 das Einrasten des Clip am Ausgang,
Figur 7 das Durchdrücken des Kniegelenks am Kniehebel,
Figur 8 die Vorderansicht des Clip in der Applikatorspitze,
Figur 9 den Applikator.

### Vorbereitung des Applikators:

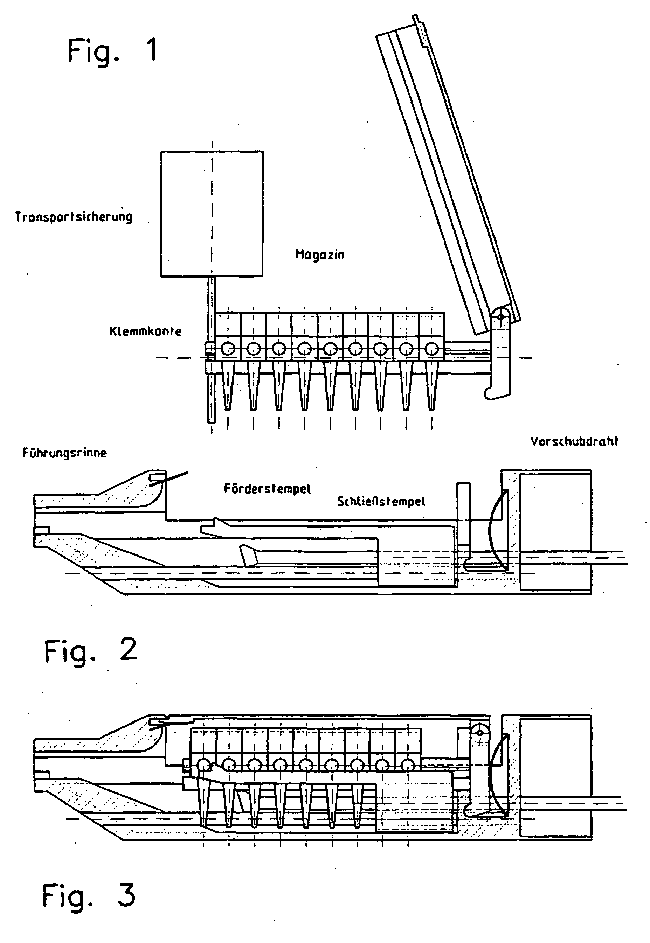

Das mit Clips gefüllte Magazin (Figur 1) wird in den Magazinschacht (Figur 2) der Applikatorspitze geschoben, bis es einrastet und über die gekrümmte Bandfeder in der Einrastnut in Position gedrückt wird. Jetzt wird die Transportsicherung (Figur 1) gezogen und der Magazindeckel geschlossen (Figur 3). Mit Hilfe einer kleinen axialen Schubbewegung arretiert der Deckel in der Wand.

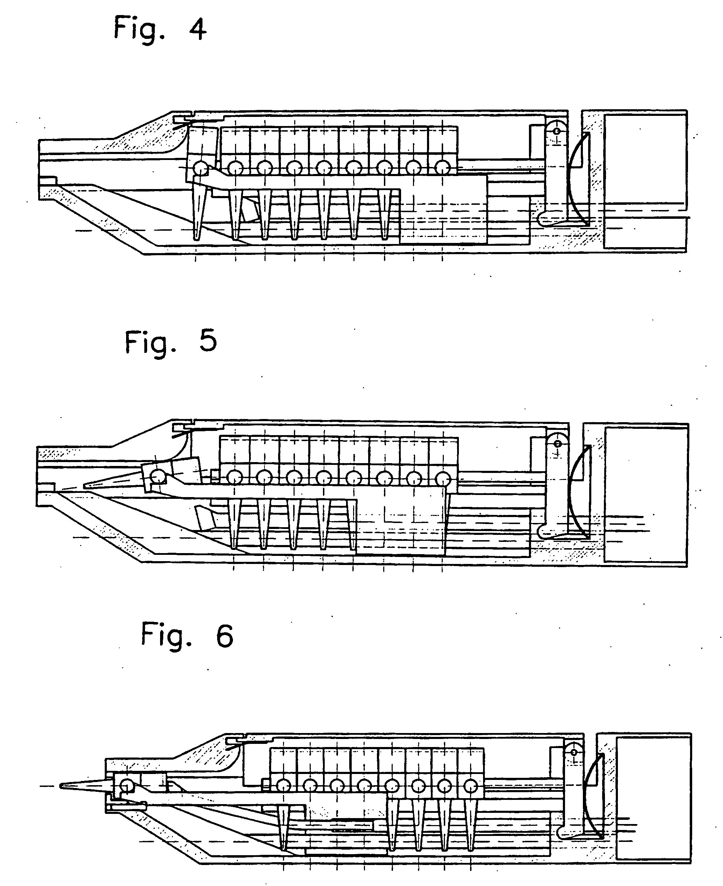

Nach dem Einlegen des Magazins befinden sich die Clips, hängend aufgereiht, in der Ausgangsposition im Magazin (Figur 1, 3 und 8). Die beiden Zähne des Förderstempels greifen links und rechtes die beiden zylindrischen Fortsätze an der Schulter des vordersten Clips im Magazin (Figur 4) und beginnen ihn abzuheben. Die Clips im Magazin sind durch eine Feder vorgespannt und werden durch eine Klemm- oder Anschlagkante am freien Ende der Stange des Magazins am Herausfallen gehindert. Der Clip wird durch den Förderstempel über die Klemmkante aus dem Magazin in die Führungsrinne geschoben und gleichzeitig mit dem weiteren Vorfahren durch die Rundung in der Wand unterhalb des Verschlusses des Magazindeckels um 90° gedreht (Figur 5).

Während dieser Bewegung aus dem Magazin heraus, wird der Clip an der Seite durch die beiden zylindrischen Fortsätze an den Schultern in Rinnen durch das umgebende Gehäuse mit nach vorne zeigenden Zähnen zwangsweise geführt.

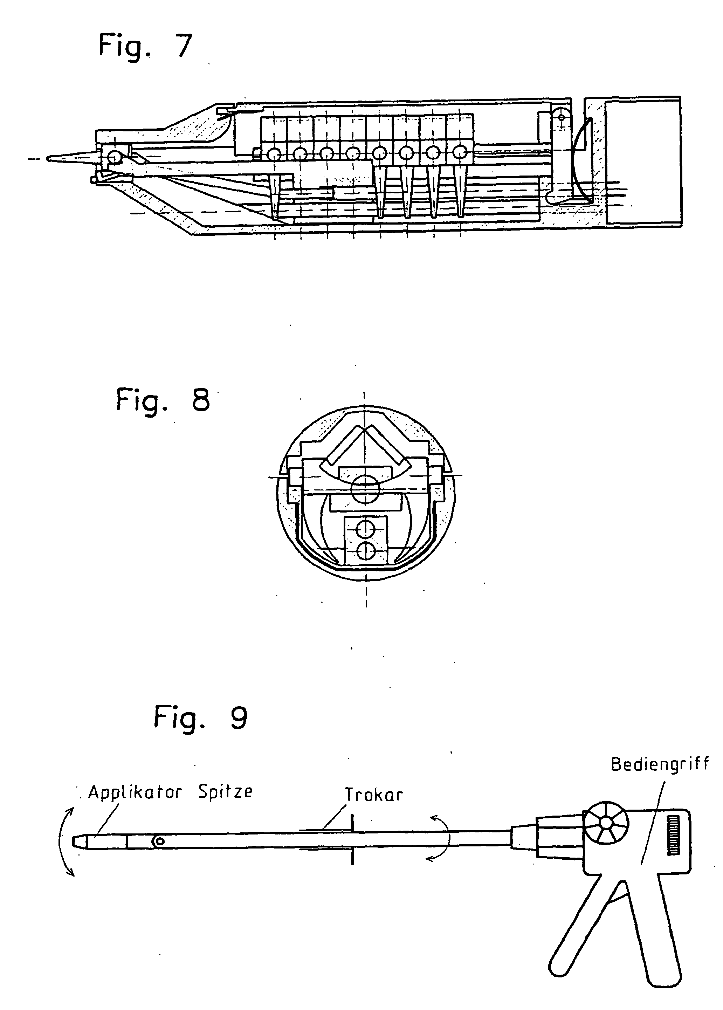

Die weitere Vorwärtsbewegung des Förderstempels befördert den Clip an Stützfedern vorbei in den beiden Rinnen an den Operationsausgang der Applikatorspitze. Die Stützfedern verhindern ein Zurückrutschen des Clip. Am Ausgang der Applikatorspitze sind Rückhalteklammern (Figuren 6 und 7) angebracht, die den Clip gegen vorzeitiges Herausfallen in der Endlage vor dem Einsetzen ins Gewebe sichern. An diesem Anschlag hat der Förderstempel seine maximale axiale Auslenkung erreicht. Der Förderstempel ist über eine Feder mit dem Schubdraht verbunden, so daß er von einer weiteren Vorwärtsbewegung des Schubdrahtes ab dieser Position abgekoppelt bleibt.

In dieser Position wird der Handgriff am Bedienteil nicht weiter gespannt und verharrt in dieser Lage.

Der Applikator wird an der vorgesehen, geeigneten Stelle im Gewebe positioniert. Bei entsprechender Bearbeitungsrichtung dringen die Spitzen des Clips im Falle der Leistenbruchoperation durch das Netz in die obere Hautschicht ein.

Bis jetzt sind die Spitzen des Clips erst in die Haut eingedrungen, sind jedoch noch in der Applikatorspitze arretiert. Wird der Handgriff weiter gezogen (Figur 7), wird das Kniegelenk am Kniehebel mehr und mehr durchgezogen, wodurch die Schulter über die beiden Gelenke, die den Kniehebel mit der Schulter koppeln, gekrümmt wird und sich die Spitzen der Zähne oder Krallen des Clips im durchdrungenen Gewebe schließen.

Drückt man den Handgriff bis in seine Endstellung, klappt die Rückhalteklammer weg und der Clip wird freigegeben und man kann die Applikatorspitze zurück ziehen(Figur 7).

Wird der Handgriff am Bedienteil des Applikators (Figur 9) losgelassen, zieht sich der Schubdraht mit den beiden Stempeln an seinem distalen Ende in die Ausgangsstellung über Federkraft zurück. Die beiden Mitnehmerzähne am Förderstempel heben den vordersten Clip an. Mit dem ersten Clip werden auch alle noch im Magazin verbliebenen Clips angehoben, wobei die Förderfeder des Magazins verhindert, daß die Clips nach hinten geschoben werden. Sobald der Mitnehmerzahn den ersten Clip passiert hat, klappt das Magazin wieder in seine Normalstellung zurück (Figur 3). Nun liegt der erste Clip wieder vor dem Mitnehmerzahn und der Förderstempel hat ebenfalls seine Ausgangsstellung erreicht. Jetzt kann ein neuer Clip aus dem Magazin entnommen und gesetzt werden.

## Patentansprüche

1. Applikatorspitze eines chirurgischen Applikators zum Setzen von Clips/Klammern für die Verbindung von Gewebe, bestehend aus:
- einem in einen dafür vorgesehenen Magazinschacht einsetzbaren Magazin, in dem die Clips/Klammern gestapelt sind
- einem Vorschubmechanismus, der aus dem eingesetzten Magazin sukzessive Clips/Klammern zum Setzen ausklinkt, und
- einem Schliessmechanismus, der den/die ausgeklinkte(n) Clip/Klammer Schliesst,
**dadurch gekennzeichnet, daß** Clips, die aus einer zunächst geraden, mit einem Kniegelenk überbrückten Schulter bestehen, von der zwei spiegelbildlich zueinander gekrümmte Zähne abstehen und auf einer Stapelstange mit ihrem unteren Schulterbereich hängend und vorgespannt bis zum freien Ende aneinander gereiht aufliegen,
die Stapelstange mit ihrer hinteren Stirnseite an einer Basisplatte verankert ist, an der ein Klappdeckel drehbar gelagert ist, und eine herausnehmbare Transportsicherung am freien Ende der Stapelstange gesteckt ist, die nach dem Einsetzen des Magazins in den Magazinschacht ziebar ist,
die Applikatorspitze aus einem Magazinschacht besteht, in dessen hinteren Bereich senkrecht zur Applikatorachse eine Ausnehmung zum Einrasten der Basisplatte des Magazins ist und sich in dieser ein Federbügel befindet, der die Basisplatte in Position drückt,
in den Magazinschacht zwei axial bewegliche, vom Bediengriff des Applikators aus, über einen Schubdraht betriebene Stempel, der Förder- und Schließstempel, ragen, wobei mit dem ersten Stempel der im Magazin in Front liegende Clip unter gleichzeitiger Ausführung einer 90°-Drehung durch eine dortige Abrundung der Wand im Auftreffbereich der Schulter des vorgeschobenen Clip von einer Klemmkante am freien Ende der Stapelstange abhebt und in einer Führungsrinne durch das Gehäuse geführt an den Ausgang der Applikatorspitze in Position schiebbar ist, wobei er ab da vom Schubdraht unter Beibehaltung der Lage abkoppelt, um danach mit dem zweiten Stempel über das Durchdrücken des Kniegelenks unter Zangenbildung in das Gewebe eingedrückt und dort verankert zu werden.

2. Applikatorspitze nach Anspruch 1,
**dadurch gekennzeichnet, daß** in der Wand der Applikatorspitze eine Ausnehmung ist, in die eine Zunge an der freien Stirnseite des Klappdeckels durch eine kurze axiale Bewegung einrastet.

3. Applikatorspitze nach Anspruch 2, **dadurch gekennzeichnet, daß** der Förderstempel zwei axiale Mitnahmezähne hat, die spiegelbildlich zueinander liegen, an ihrem jeweiligen freien Endbereich parallel zur Applikatorachse abknicken und dort eine Ausnehmung haben, in die sich ein zylindrischer Fortsatz an den beiden Schulterseiten des zur Entnahme bereiten Clip beim Abheben vom Magazin einlegt.

4. Applikatorspitze nach Anspruch 3, **dadurch gekennzeichnet, daß** sich in der Führungsrinne Federeinrichtungen befinden, die eine Vorwärstbewegung des geführten Clip zulassen, nicht jedoch eine Rückwärtsbewegung.

5. Applikatorspitze nach Anspruch 4, **dadurch gekennzeichnet, daß** an ihrem Ausgang mindestens eine Rückhalteklammer angebracht ist, die den in Position geschobenen Clip hält.

6. Applikatorspitze nach Anspruch 5, **dadurch gekennzeichnet, daß** sich in ihrem vorderen Bereich eine Rampe befindet, über die die Stirn des Schließstempels während des In-Position-Schiebens des Clips anhebbar ist und schließlich an das noch abgewinkelte Kniegelenk des Kniehebels anlegt.

## Revendications

1. Pointe d'applicateur d'un applicateur chirurgical pour la pose de clips/agrafes pour relier des tissus, qui consiste en:
- un magasin que l'on peut introduire dans une gaine de magasin prévue à cet effet et dans lequel les clips/agrafes sont empilés,
- un mécanisme d'avancement qui déclenche successivement les clips/agrafes à poser à partir du magasin introduit,
**caractérisé en ce que**
- les dips composés d'une épaule initialement droite, raccordée par une genouillère dont s'écartent deux dents courbées inversement l'une par rapport à l'autre et qui, suspendus et précontraints, s'appuient sur une tige d'empilage à leur section d'épaule inférieure, enfilés jusqu'à leur extrémité libre.
- la tige d'empilage est ancrée par son front arrière à une plaque de base dans laquelle est logé un couvercle orientable à charnière, un circlip de transport enlevable étant fiché à l'extrémité libre de la tige d'empilage qui peut être retiré après l'introduction du magasin dans la gaine de magasin,
- que la pointe d'applicateur consiste en une gaine de magasin qui comprend dans sa section arrière un creux perpendiculaire à l'axe de l'applicateur pour enclencher la plaque de base du magasin, la pointe contenant une bride de ressort qui presse la plaque de base dans sa position,
- deux poinçons, le poinçon de transfert et le poinçon de fermeture, orientables axialement, commandés à travers un fil de poussée à partir de la manette de service de l'applicateur, pénètrent dans la gaine de magasin, le premier poinçon provoquant que le clip situé sur le front du magasin s'enlève d'un bord de serrage à l'extrémité libre de la tige d'empilage en effectuant simultanément un rotation de 90° due à un arrondissement de la paroi dans la section d'impact de l'épaule du clip avancé, d'où il peut être poussé à la sortie de la pointe d'applicateur, conduit à travers la cage dans un conduit de guidage, d'où il se découple du fil de poussée tout en maintenant son orientation, ensuite, moyennant le deuxième poinçon, mécanisme de fermeture qui ferme le clip/l'agrafe déclenché(e), le clip/l'agrafe est enfoncé(e) dans le tissus et ancré(e) dans celui-ci, en tendant la genouillère, fermant ainsi une pince.

2. Pointe d'applicateur selon la revendication 1,
**caractérisée en ce que** dans la paroi de la pointe d'applicateur, il se trouve un creux dans lequel s'enclenche une lame du coté frontal libre du couvercle à charnière en effectuant un mouvement axial court.

3. Pointe d'applicateur selon la revendication 2, **caractérisée en ce que** le poinçon de transfert possède deux dents d'entraînement axiales, placées en position inversée l'une par rapport à l'autre, qui plient parallèlement à l'axe de l'applicateur à leur extrémité libre respective, où elles possèdent un creux dans lequel, au moment de l'enlèvement du magasin, un prolongement cylindrique s'insère dans les deux cotés d'épaule du clip prêt à être retiré.

4. Pointe d'applicateur selon la revendication 3, **caractérisée en ce que** des dispositifs à ressort se trouvent dans le conduit de guidage, qui admettent l'avancement du clip guidé, mais pas un mouvement en retour.

5. Pointe d'applicateur selon la revendication 4, **caractérisée en ce qu'**au moins une agrafe de retenue est disposée à sa sortie, qui tient le clip poussé dans sa position.

6. Pointe d'applicateur selon la revendication 5, **caractérisée en ce que** dans la section avant, il se trouve une rampe à travers de laquelle le front du poinçon de fermeture peut être enlevé pendant que le clip est poussé dans sa position et s'applique finalement à la genouillère encore pliée du levier à genouillère.

## Claims

1. Applicator tip of a surgical applicator for the setting of clips / clamps for tissue connection, consisting of
- a magazine, in which the dips / clamps are stacked and which is to be inserted into the magazine shaft provided,
- a feeding mechanism, by means of which dips / damps are removed successively from the inserted magazine for setting, and
**characterized by** the clips initially having a straight shoulder that is bridged by a knee joint with two teeth that are curved towards each other in a laterally reversed manner and stick out from this shoulder and the dips being arranged in a row on a stacking pin, such that they hang with their lower shoulder part and are pre-stressed up to the free end,
the rear surface of the stacking pin being fixed to a base plate that is provided with a pivoted hinged lid and a removable transportation lock being located at the free end of the stacking pin, which can be drawn upon the insertion of the magazine into the magazine shaft, and the applicator tip consisting of a magazine shaft, the rear area of which is provided with a recess vertical to the applicator axis for catching the base plate of the magazine, with this base plate containing a spring buckle that pushes it into the correct position, and
two dies operated by a pushing wire, the feeding and the dosing die, extending into the magazine shaft and being axially movable from the operation handle of the applicator, with the first die lifting off the first dip in the magazine from the edge at the free end of the stacking pin and rotating this clip by 90° with the help of the rounded-off wall in the contact area of the dip shoulder, and this dip being moved in a guide groove through the housing towards the exit of the applicator tip, where it is decoupled from the pushing wire, while maintaining its position, and the second die representing a mechanism that closes the released clip(s) / damp(s) by pulling the knee joint, as a result of which the dip is pressed into the tissue and fixed there like a pair of pliers.

2. Applicator tip according to Claim 1,
**characterized by** the wall of the applicator tip being provided with a recess for catching the tongue at the free end of the hinged lid by a short axial movement.

3. Applicator tip according to Claim 2,
**characterized by** the feeding die being equipped with two axial pushers that are arranged in a laterally reversed manner and that bend off parallel to the applicator axis at their free ends, where they are equipped with a recess for catching the cylindrical extension that is located on both shoulder sides of the clip ready for withdrawal and lift-off from the magazine.

4. Applicator tip according to Claim 3,
**characterized by** the guide groove being equipped with spring units that allow for a forward, but not for a backward movement of the guided dip.

5. Applicator tip according to Claim 4,
**characterized by** at least one retaining dip being installed at the tip exit, by means of which the dip that has been shifted into position is retained.

6. Applicator tip according to Claim 5,
**characterized by** its front section being equipped with a ramp, via which the front of the dosing die can be lifted off while shifting the clip into its position and moved towards the still knee-shaped joint of the lever.
